# FASCICULE DE BREVET EUROPEEN

(11) **EP 1 810 961 B1**
(45) Date de publication et mention de la délivrance du brevet: **29.12.2010**
(21) Numéro de dépôt: 07290062.4
(22) Date de dépôt: 15.01.2007
(51) Int. Cl.: C07C 67/475, C07C 69/593, C07C 51/347, C07C 57/13, C07C 6/04, C07C 11/02

(54) **Procédé de co-production d'oléfines et de diesters ou de diacides par homométathèse de corps gras insaturés dans des liquides ioniques non-aqueux**
Verfahren zur gleichzeitigen Produktion von Olefinen und Diestern oder Disäuren mittels Homometathese von ungesättigten Fettkörpern in nichtwässrigen, ionischen Flüssigkeiten
Method of co-producing olefins and diesters or diacids by homometathesis of unsaturated fatty bodies in non-aqueous ionic liquids

(30) Priorité: 24.01.2006 FR 0600645
(43) Date de publication de la demande: 25.07.2007
(73) Titulaire: Institut Français du Pétrole, 92852 Rueil Malmaison Cedex (FR)
(72) Inventeur: Olivier-Bourbigou, Hélène, 69320 Saint Genis Laval (FR); Hillion, Gérard, 95220 Herblay (FR); Vallee, Christophe, 38600 Fontaine (FR)

(56) Documents cités:
- EP-A- 1 035 093
- WO-A-96/04289
- WO-A-99/51344
- US-A- 5 675 051
- OLIVIER-BOURBIGOU H ET AL: "Ionic liquids: perspectives for organic and catalytic reactions" JOURNAL OF MOLECULAR CATALYSIS. A, CHEMICAL, ELSEVIER, AMSTERDAM, NL, 2002, pages 419-437, XP002274532 ISSN: 1381-1169

## Description

### Domaine de l'invention

L'invention concerne la coproduction d'oléfines et de diesters ou de diacides par homométathèse de corps gras insaturés, en présence d'un catalyseur et d'au moins un liquide ionique non-aqueux.

### Arrière-plan technologique

La réaction de métathèse des oléfines est une réaction bien connue de la chimie organique. Cette réaction, qui s'effectue en présence d'un système catalytique approprié, consiste en l'échange de groupes alkylidènes entre deux oléfines selon les équations suivantes :
1. Premier cas, dit de "métathèse croisée" ou "cross-metathesis" (c'est-à-dire de métathèse entre deux oléfines différentes) : 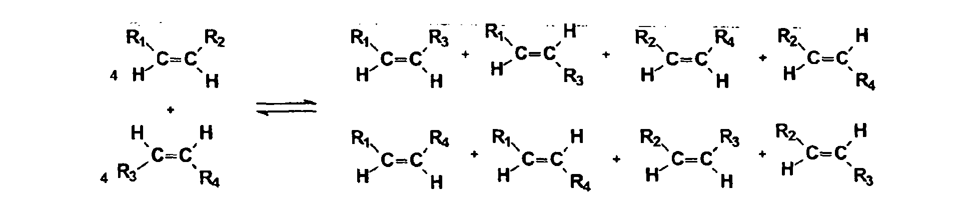
2. Second cas, dit de "self métathèse" ou "homométathèse" (c'est à dire de métathèse d'une molécule d'oléfine sur une molécule de la même oléfine):

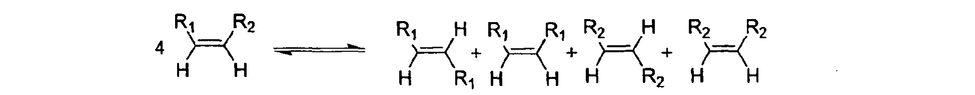

La réaction de métathèse des oléfines est une réaction équilibrée. Elle peut se produire en présence d'une grande variété de catalyseurs, le plus souvent à base de métaux de transition des groupes IVA à VIII, parmi lesquels le tungstène, le molybdène, le rhénium et le ruthénium, soit en phase homogène, soit en phase hétérogène. De nombreuses revues et ouvrages scientifiques traitent de cet aspect. On citera par exemple :
- K. J. Ivin and J. C. Mol dans "Olefin Metathesis and Metathesis Polymerization", San Diego, Academic Press (1997) ;
- "Handbook of Metathesis" R.H. Grubbs (Ed) Wiley-VCH, Weinheim (2003) ;
- J .C. Mol "Industrial Applications of Olefin Metathesis" J. Mol. Catal. 213, 39 (2004) ;
- D. Séméril and P.H. Dixneuf, dans "Novel Metathesis Chemistry: Well-Defined Initiator Systems for Specialty Chemical Synthesis, Tailored Polymers and Advanced Material Applications" Y. Imamoglu and L. Bencze (Eds) Kluwer Academic Publishers, The Netherlands (2003) 1-21.

Si l'oléfine est un ester d'acide gras insaturé, représenté par exemple par l'oléate de méthyle, la réaction conduit à l'obtention d'une oléfine et d'un diester insaturé. La réaction peut s'écrire :

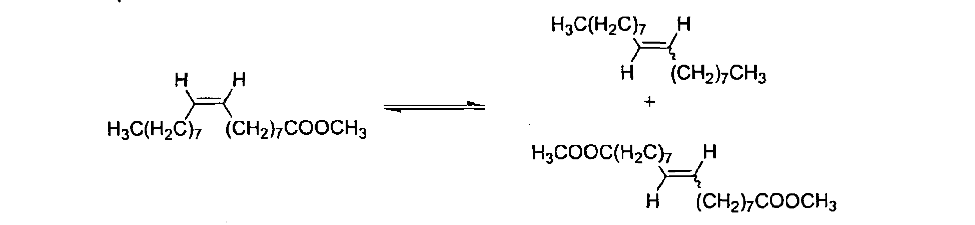

Cette réaction est potentiellement d'un grand intérêt, car elle permet d'envisager la fabrication à partir d'une matière première pour l'essentiel d'origine végétale ou animale, donc renouvelable, des produits tels qu'un diester insaturé et qu'une oléfine longue. Dans le cas particulier envisagé, le diester insaturé est le diester méthylique de l'acide octadéc-9-ène-1,18-dioïque, qui possède des potentialités pour la fabrication de certains polymères (polyesters, polyamides), et l'oléfine longue est l'octadéc-9-ène, qui peut être dimérisé et hydrogéné pour conduire au 10,11-dioctyleicosane qui possède des propriétés intéressantes en tant que lubrifiant.

Cette réaction d'homométathèse appliquée à des esters d'acides gras a été reportée dans de nombreuses publications ou revues scientifiques. À titre d'exemples, on peut citer, parmi les références les plus récentes :
- "Application of Olefin Metathesis in Oleochemistry: an Example of Green Chemistry" par J. C. Mol , Green Chemistry, 4, 5-13 (2002) ;
- "The Metathesis of Polyunsaturated Fatty Esters Using the Homogeneous W(O-2,6-C6H3X2)2Cl4/Me4Sn Catalytic Systems" par B.B. Marvey et coll. J. Mol. Catal. 213, 151-157 (2004) ;
- "Technological and Economical Aspects of the Metathesis of Unsaturated Esters" par M. Sibeijn et coll. JAOCS, 71, 6 (1994) ;
- "Polymer and Surfactants on the Basis of Renewable Resources" par S. Warwel et coll. Chemosphere 43, 39-48 (2001) ;
- "Catalysis Metathesis of Unsaturated Fatty Acid Esters and Oils" par J.C. Mol, Topics in Catalysis, 27, 1, (2004).

Différents types de catalyseurs ont été décrits pour réaliser une telle transformation. Les premiers systèmes sont homogènes, à base de tungstène et de tétraalkylétains, par exemple WCl₆/SnMe₄. Puis les systèmes sont hétérogènes à base de rhénium activé par des tétraaklylétains. Cependant, ces systèmes ont l'inconvénient de mettre en oeuvre des co-catalyseurs, en général à base d'étain qui peuvent contaminer les produits de la réaction. Plus récemment, les systèmes homogènes "bien définis", n'utilisant pas de co-catalyseur, et à base de métal-carbènes (M=C) ont été décrits, le métal étant le tungstène ou le molybdène. Cependant, la principale difficulté rencontrée avec l'ensemble de ces systèmes reste leur faible compatibilité avec les groupes fonctionnels tels que les acides ou les esters tels que ceux présents dans les huiles végétales. Cela entraîne en général une faible activité et une désactivation rapide de ces systèmes catalytiques.

Les complexes à base de ruthénium s'avèrent rapidement très intéressants en raison de leur tolérance vis-à-vis d'une large gamme de groupes fonctionnels. Cette propriété, couplée avec une activité souvent élevée, explique leur développement important dans le domaine de la synthèse des polymères et pour la synthèse organique.

Leur utilisation pour catalyser la métathèse des huiles végétales a été largement moins étudiée. On peut trouver néanmoins les références suivantes :
- La demande internationale WO-A-96/04289 (R. Grubbs et al.), qui décrit l'homométathèse de l'oléate de méthyle et de l'acide oléique avec des complexes de type 1 (Figure 1 ci-dessous). La réaction conduit à un mélange à l'équilibre comprenant une oléfine et un diester ou un diacide insaturés.
- La demande internationale WO-A-99/51344 (W. Herrmann et al.), le brevet US 6 635 768 (Herrmann et al.) et la demande de brevet US 2004/0095792A1 (Herrmann et al.), qui décrivent l'utilisation de complexes analogues de type 2 (Figure 1 ci-dessous) pour catalyser l'homométathèse de l'oléate de méthyle et la métathèse de l'oléate de méthyle avec l'octène-1. Ces derniers complexes peuvent être plus actifs mais montrent également une activité isomérisante de la double liaison.
- La demande internationale WO-A-02/076920 (Newman et al.), qui décrit la mise en oeuvre de complexes du ruthénium de type 3 (Figure 1 ci-dessous) en milieu homogène ou supporté sur polymères, par exemple de type polystyrène. La particularité de ces complexes par rapport aux précédents est qu'ils portent un ligand chélate. Il apparaît clairement que l'immobilisation du complexe sur un support solide diminue considérablement l'activité du système.

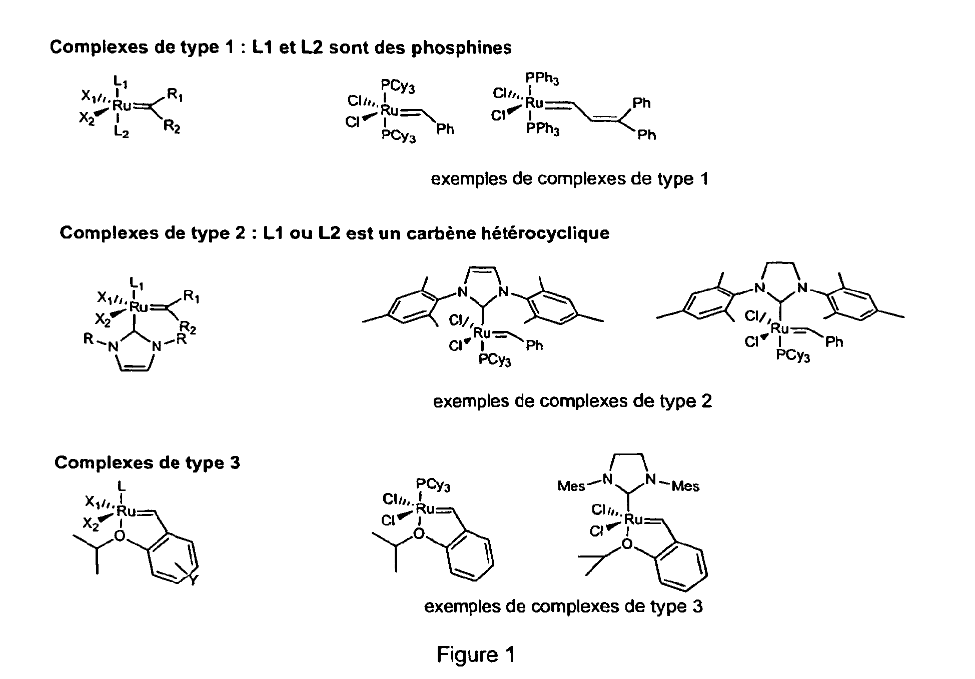

Ainsi, l'une des difficultés principales de ces systèmes à base de ruthénium est leur trop faible durée de vie. Leur mise en oeuvre en phase homogène donne les complexes les plus actifs, mais pose le problème de la séparation des produits de la réaction et de leur recyclage.

Une approche intéressante consiste à immobiliser le catalyseur dans une phase liquide (solvant), de laquelle on peut aisément séparer les produits, soit par distillation, soit par décantation si les produits sont peu miscibles avec le solvant.

Les liquides ioniques non-aqueux de formule générale Q⁺A⁻ s'avèrent être des solvants particulièrement intéressants pour cette application. Ils présentent une très faible tension de vapeur (ne distillent pas) et des propriétés physico-chimiques modulables en fonction de l'anion et du cation qui les composent (voir par exemple H. Olivier-Bourbigou, L. Magna, J. Mol. Catal. A, Chem. 2002, vol. 182, p. 419).

L'immobilisation des catalyseurs à base de ruthénium dans les liquides ioniques a été décrite mais peu de littérature existe dans ce domaine. On peut citer par exemple le brevet EP-B-1 035 093. Cependant, les applications décrites ne concernent que des cas de métathèse par fermeture ou ouverture de cycle (RCM ou ROMP).

D'autre part, un des problèmes principaux de cette réaction d'homométathèse réside dans les rendements de conversion. Cette réaction étant une réaction équilibrée, le rendement maximal en produits obtenus est de 50 %. Une solution qui permettrait d'augmenter la conversion en déplaçant l'équilibre est donc particulièrement recherchée.

L'homométathèse des corps gras insaturés n'est pas décrite dans les liquides ioniques. Comme les variations d'enthalpie associées à ce type de réaction sont très faibles, le résultat à l'équilibre thermodynamique est proche d'une distribution statistique des groupements alkylidènes. Ainsi, pour l'homométathèse de l'oléate de méthyle, la composition du mélange à l'équilibre est proche de 50 % de produits de départ, de 25 % de l'oléfine longue en C18 et de 25 % de diester. En vue d'une application industrielle, il est donc nécessaire de recycler le réactif non converti après séparation des produits de la réaction.

Le développement d'un procédé d'homométathèse des corps gras insaturés économiquement viable implique donc :
- de mettre au point un catalyseur stable et peu isomérisant de la double liaison ;
- un procédé dans lequel l'oléfine longue co-produite durant la réaction soit sélectivement extraite du milieu réactionnel, ceci afin de permettre le déplacement de l'équilibre dans le sens de la formation de l'oléfine longue et du diester ou du diacide ;
- un procédé dans lequel le catalyseur est recyclable et réutilisable.

### Objet de l'invention

L'invention a pour objet un procédé impliquant l'homométathèse de corps gras insaturés, en présence d'un catalyseur comprenant par exemple au moins un composé du ruthénium et en présence d'au moins un liquide ionique non-aqueux.

L'invention a plus particulièrement pour objet un procédé d'homométathèse de corps gras choisis parmi les esters de monoalcools des huiles de tournesol oléiques et des huiles de colza oléiques et les mélanges d'acides correspondants.

Dans ce nouveau procédé, le catalyseur (par exemple à base de complexe du ruthénium) est immobilisé et stabilisé dans le liquide ionique non-aqueux, dans lequel les oléfines produites sont très peu miscibles. Celles-ci sont donc extraites, pendant la réaction, et dès leur formation, dans une deuxième phase.

Dans ce nouveau procédé, les produits de la réaction peuvent être séparés aisément du liquide ionique contenant le catalyseur soit par distillation du fait de la non-volatilité du liquide ionique, soit par décantation du fait de la faible solubilité des oléfines formées dans le liquide ionique. Le catalyseur reste immobilisé et stabilisé dans le liquide ionique. Ce dernier contenant le catalyseur peut être recyclé et réutilisé.

Ce procédé est utilisé pour obtenir des compositions particulières de produits, qui seront séparés en plusieurs fractions distinctes ayant chacune un usage différent.

### Description détaillée de l'invention

### La charge

Le procédé de métathèse de l'invention s'adresse à tout corps gras comprenant au moins un monoacide carboxylique ou un monoester possédant de 12 à 22 atomes de carbone et comportant au moins une insaturation éthylénique.

L'obtention d'acides gras, qui est bien connue de l'homme du métier, est le plus souvent réalisée par hydrolyse en milieu acide sur des huiles ou des graisses.

Les esters d'acides gras peuvent être obtenus soit par estérification des acides gras ou par transestérification directe des huiles (ou triglycérides) avec un composé aliphatique saturé monohydroxylé, comme par exemple le méthanol, l'éthanol, le propanol ou plus généralement tout monoalcool renfermant de 1 à 8 atomes de carbone.

Les acides gras sont les composants majoritaires des huiles d'origines végétale ou animale. Ils sont rarement obtenus purs à l'état naturel et sont toujours constitués de mélanges de plusieurs acides gras.

Les principaux acides gras monoinsaturés que l'on rencontre naturellement dans les huiles sont souvent porteurs de l'insaturation sous forme cis et en position Δ9 [position de l'insaturation comptée à partir du groupement carboxylique].

Dans cette famille, on rencontre par exemple : l'acide lauroléique (acide dodécèn-9c oïque), l'acide myristoléique (acide tétradécèn-9c oïque), l'acide palmitoléique (acide hexadécèn-9c oïque), l'acide oléique (acide octadécèn-9c oïque), l'acide gadoléique (acide eicosèn-9c oïque), l'acide cétoléique (acide docosèn-9c oïque).

On trouve également, à l'état naturel, des isomères de position de l'insaturation de l'acide oléique comme l'acide cis-vaccénique (acide octadécèn-11c oïque) ainsi que l'acide pétrosélinique (acide octadécèn 6c oïque), d'autres acides gras où l'insaturation est en (n-9), [position de l'insaturation comptée à partir du groupement méthyl terminal de la chaîne grasse], comme par exemple, l'acide hypogéïque (acide hexadécèn-7c oïque), l'acide gondoïque (acide eicosèn-11c oïque), l'acide érucique (acide docosèn-13c oïque), l'acide nervonique (acide tétracosèn-15c oïque).

La plupart de ces acides sont des composés mineurs de certaines huiles ou présents à des teneurs plus élevées dans des graines de plantes dont la culture reste confidentielle ou très limitée.

On trouve également à l'état naturel des isomères trans d'acides gras monoinsaturés. On peut citer l'acide vaccénique (acide octadécèn-11t oïque).

L'hydrogénation partielle de corps gras polyinsaturés s'accompagne toujours d'isomérisations cis-trans. Ces isomérisations peuvent affectées également toutes les monooléfines présentes. On peut citer en particulier l'acide élaïdique (9t) et brassidique (13t) qui sont respectivement les isomères trans de l'acide oléique et de l'acide érucique.

Il n'existe pas à l'état naturel d'acide monoinsaturé à double liaison terminale. Cependant, l'acide undécylènique (acide undécèn-10 oïque), qui résulte du craquage de l'acide ricinoléique, qui est un produit industriel entrant dans la synthèse du nylon-11 (Rilsan®) peut être un intermédiaire intéressant en homométathèse, car de grande pureté, et susceptible de donner majoritairement, sous la forme ester méthylique, un diester monoinsaturé en C20 et de l'éthylène.

On peut également citer les acides gras à fonctions secondaires oxygénées, avec principalement des fonctions alcools, dont le principal est l'acide ricinoléique (acide hydroxy-12L octadécèn-9c oïque), constituant principal de l'huile de ricin.

Après homométathèse, dans ce cas, il n'y a plus de monooléfines fabriquées mais un diol oléfinique en C18 avec du diester monoinsaturé en C20.

Comme il n'existe pas dans la nature de corps gras d'origine végétale ou animale dont les chaînes grasses seraient exclusivement constituées de chaînes oléiques, l'obtention d'un ester de l'acide oléique pur nécessite donc de recourir à une opération de séparation et de purification faisant le plus souvent appel à la distillation ou à la cristallisation dans des conditions difficiles et donc coûteuses.

Il existe aujourd'hui des huiles dites "oléiques" issues de variétés de tournesol et de colza. Les teneurs en acide oléique dépassent souvent 80 %. Par contre la teneur en acide linoléique (acide octadécadièn-9c12c oïque) peut atteindre 10 à 12 % et pénaliser la qualité des produits résultant de la réaction d'homométathèse en fabriquant un nombre important d'isomères de diacides et de composés mono- et polyoléfiniques.

Une autre variante permettant d'obtenir des matières premières enrichies en acides gras monoinsaturés de type oléique est l'hydrogénation sélective de mélanges d'acide gras contenant des acides polyinsaturés en C18. Dans ce cas, le produit obtenu est composé d'isomères trans et d'isomères de position de la double liaison. Pour une chaîne grasse diénique, cas de l'acide linoléique (acide octadécadièn-9c,12c oïque), on obtient, après une étape d'hydrogénation contrôlée, un mélange d'isomères cis et trans et d'isomères de positions (Δ 9, 10, 11 et 12).

L'hydrogénation sélective de certains corps gras pourrait donc permettre d'enrichir la teneur en chaînes grasses oléiques de certains mélanges d'acides gras et d'élargir ainsi la gamme des matières premières susceptibles d'être utilisées en homométathèse.

Par exemple, l'hydrogénation sélective d'huiles de tournesol oléique ou de colza non oléique, dont la distribution en acides gras est la suivante: acide palmitique (5 %), stéarique (2 %), oléique (59 %), linoléique (21 %), linolènique (9 %) et acides gras supérieurs en C20 et C22 (3 %), permettrait d'atteindre une composition en chaînes grasses monoinsaturées proche des 90 %, le complément étant principalement des chaînes saturées. Dans ce cas, à partir de cette matière première modifiée chimiquement, la réaction d'homométathèse conduirait principalement à la formation de deux produits, l'octadéc-9-ène et le diester (l'ester méthylique de l'acide octadéc-9-ène-1,18 dioïque).

La matière première la plus adaptée au procédé de l'invention devra donc être particulièrement riche en acide oléique ou en ses isomères, chaînes grasses porteuses d'une seule insaturation, de façon à obtenir majoritairement un mélange riche en diester de l'acide octadécè-9-ène-1, 18 dioïque et en octadéc-9-ène.

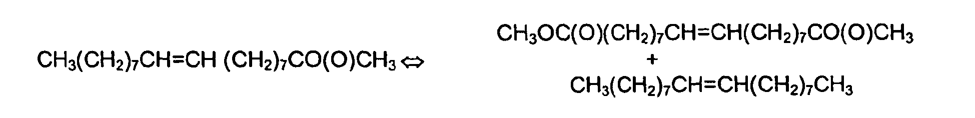

Si l'oléfine est un ester d'acide gras di-insaturé, tel qu'un ester méthylique de l'acide linoléique, la réaction de métathèse conduirait à des mono- ou poly-oléfines, des mono-esters mono- ou poly-insaturés et des diesters mono- ou poly-insaturés. La réaction peut s'écrire :

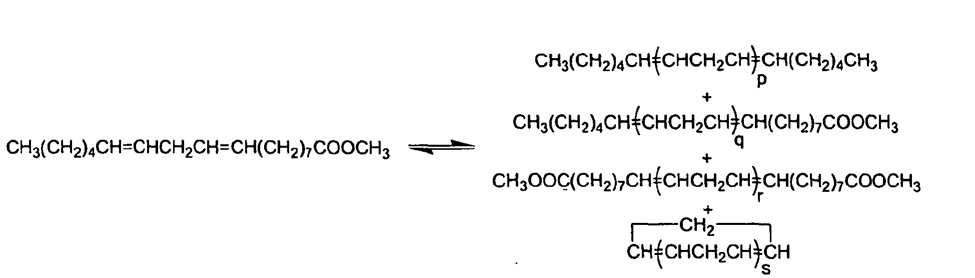

On pourrait appliquer les mêmes réactions à toutes les chaînes insaturées d'acides gras connues, par exemple les chaînes d'acide tri-insaturées de type linolènique. Le nombre de produits potentiellement possibles sera d'autant plus important que le nombre d'insaturations portées par la chaîne sera élevé.

Si l'on applique cette réaction de métathèse, non plus à une chaîne unique d'acide gras, par exemple oléique ou linoléique comme précédemment, mais à un mélange de ces chaînes d'acides gras, comme c'est le cas dans la réalité lorsque l'on a affaire à des produits d'origine végétale ou animale, on obtiendra un mélange des produits issus de l'homométathèse de chacune des chaînes grasses et de la métathèse croisée des chaînes grasses entre elles.

Dans tous les cas, les esters ou acides gras saturés présents dans les mélanges de chaînes grasses issues d'huiles de tournesol oléique et de colza oléique ne sont pas réactifs dans la réaction de métathèse et sont retrouvés à la fin de l'opération.

La nature des produits obtenus, ainsi que leur quantité dépendront donc de la composition en acides gras (nature et abondance) de la matière première grasse utilisée.

### Exemples de produits fabriqués en homométathèse sur base d'esters méthyliques d'huile de tournesol oléique.

La composition de l'ester méthylique de l'huile de tournesol oléique est la suivante:
- palmitate de méthyle : C16:0 = 3 % en masse
- stéarate de méthyle : C18:0 = 4 % en masse
- oléate de méthyle : C18:1 = 83 % en masse
- linoléate de méthyle : C18:2 =10% en masse On peut classer les produits formés en 1^{ère} réaction en quatre familles distinctes :
- les monooléfines,
- les polyoléfines,
- les monoesters et diesters insaturés et
- les esters saturés.

L'octadéc-9-ène, le dodéc-6-ène, le pentadéc-6-9-diène, l'octadec-6,9-diène et l'octadéc-6,9,12-triène sont les premières molécules d'oléfines formées. Elles peuvent réagir à leur tour sur elles-mêmes et sur l'ester méthylique mono- et di-insaturé qui n'a pas réagi pour donner d'autres molécules oléfiniques comme par exemple le tétracos-6,9,12,15,18-pentaène, l'uneicos-6,9,12,15-tétraène, l'uneicos-9,12,15, triène, etc.

Le procédé selon la présente invention peut comprendre une étape de séparation des oléfines par évaporation. En effet, on peut aisément séparer par distillation les monooléfines et les polyoléfines, comme par exemple le dodéc-6-ène, l'octadéc-9-ène, le pentadéc-6,9-diène, l'octadec-6,9-diène et l'octadéc-6,9,12,triène, du milieu réactionnel. La température d'ébullition des oléfines en C18 étant inférieure de 34°C par rapport à celle des esters méthyliques de l'acide oléique n'ayant pas réagi ou à celle des diesters en C18 fabriqués.

Dans le procédé selon la présente invention, on peut ensuite soumettre le mélange d'oléfines isolé précédemment à une distillation sélective permettant de séparer le dodéc-6-ène, l'octadéc-9-ène, le pentadéc-6,9-diène, l'octadec-6,9-diène et l'octadéc-6,9,12-triène.

Les oléfines possédant plus de 18 atomes de carbone ne pourront être séparées par cette technique étant donné leur point d'ébullition trop proche de celui des esters oléiques, des acides gras saturés et des diesters fabriqués.

Après évaporation de la fraction oléfinique (mono- et dioléfines), le reste du milieu réactionnel peut être de nouveau mis en réaction pour convertir les esters oléiques ou linoléiques susceptibles de réagir en homométathèse. Rappelons que les structures saturées d'esters d'acide gras ne sont pas concernées par la réaction de métathèse.

### Le liquide ionique

Le solvant ionique non-aqueux est choisi dans le groupe formé par les sels liquides qui ont pour formule générale Q⁺A⁻ dans laquelle Q⁺ représente un ammonium quaternaire, un phosphonium quaternaire, un guanidinium quaternaire et/ou un sulfonium quaternaire et A⁻ représente tout anion susceptible de former un sel liquide à basse température, c'est-à-dire en dessous de 90°C et avantageusement d'au plus 85°C, et de préférence en dessous de 50°C.

Les anions A⁻ sont de préférence choisis parmi les anions halogénures, nitrate, sulfate, alkylsulfates, phosphate, alkylphosphates, acétate, halogénoacétates, tétrafluoroborate, tétrachloroborate, hexafluorophosphate, trifluoro-tris-(pentafluoroéthyl)phosphate, hexafluoroantimonate, fluorosulfonate, alkylsulfonates (par exemple le méthylsulfonate), perfluoroalkylsulfonates (par exemple le trifluorométhylsulfonate), bis(perfluoroalkylsulfonyl) amidures (par exemple l'amidure de bis trifluorométhylsulfonyle de formule N(CF₃SO₂)₂⁻), le méthylure de tris-trifluorométhylsulfonyle de formule C(CF₃SO₂)₃⁻, le méthylure de bis-trifluorométhylsulfonyle de formule HC(CF₃SO₂)₃⁻, arènesulfonates, éventuellement substitués par des groupements halogènes ou halogénoalkyles, l'anion tétraphenylborate et les anions tétraphenylborates dont les noyaux aromatiques sont substitués, tétra-(trifluoroacétoxy)-borate, bis-(oxalato)-borate, dicyanamide, tricyanométhylure, ainsi que l'anion tétrachloroaluminate, ou les anions chlorozincates.

Dans les formules ci-après, R¹, R², R³, R⁴, R⁵ et R⁶ représentent l'hydrogène (à l'exception du cation NH₄⁺ pour NR¹R²R³R⁴⁺), de préférence un seul substituant représentant l'hydrogène, ou des radicaux hydrocarbyles ayant de 1 à 30 atomes de carbone, par exemple des groupements alkyles, saturés ou non-saturés, cycloalkyles ou aromatiques, aryles ou aralkyles, éventuellement substitués, comprenant de 1 à 30 atomes de carbone.

R¹, R², R³, R⁴, R⁵ et R⁶ peuvent également représenter des radicaux hydrocarbyles portant une ou plusieurs fonctions choisies parmi les fonctions -CO₂R, -C(O)R, -OR, -C(O)NRR', -C(O)N(R)NR'R", -NRR', -SR, -S(O)R, -S(O)₂R, -SO₃R, -CN, -N(R)P(O)R'R', -PRR', -P(O)RR', -P(OR)(OR'), -P(O)(OR)(OR') dans lesquelles R, R' et R", identiques ou différents, représentent chacun l'hydrogène ou des radicaux hydrocarbyles ayant de 1 à 30 atomes de carbone.

Les cations Q⁺ ammonium et/ou phosphonium quaternaires répondent de préférence à l'une des formules générales NR¹R²R³R⁴⁺ et PR¹R²R³R⁴⁺, ou à l'une des formules générales R¹R²N=CR³R⁴⁺ et R¹R²P=CR³R⁴⁺ dans lesquelles R¹, R², R³ et R⁴, identiques ou différents, sont définis comme précédemment.

Les cations ammonium et/ou phosphonium quaternaires peuvent également être dérivés d'hétérocycles azotés et/ou phosphorés comportant 1, 2 ou 3 atomes d'azote et/ou de phosphore, de formules générales : 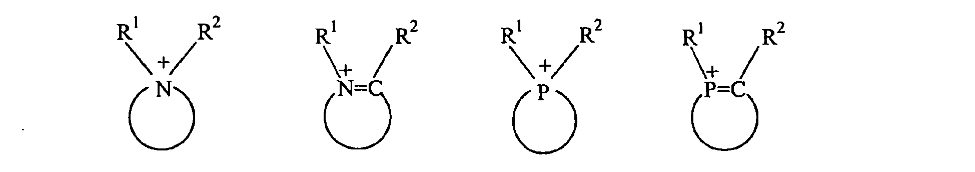 dans lesquelles les cycles sont constitués de 4 à 10 atomes, de préférence de 5 à 6 atomes, et R¹ et R², identiques ou différents, sont définis comme précédemment.

Le cation ammonium ou phosphonium quaternaire peut en outre répondre à l'une des formules générales :

R¹R²⁺N=CR³-R⁷-R³C=N⁺R¹R² et R¹R²⁺P=CR³-R⁷-R³C=P⁺R¹R²

dans lesquelles R¹, R² et R³, identiques ou différents, sont définis comme précédemment, et R⁷ représente un radical alkylène ou phénylène.

Parmi les groupements R¹, R², R³ et R⁴, on mentionnera les radicaux méthyle, éthyle, propyle, isopropyle, butyle primaire, butyle secondaire, butyle tertiaire, amyle, phényle ou benzyle ; R⁷ pourra être un groupement méthylène, éthylène, propylène ou phénylène.

De manière préférée, le cation ammonium et/ou phosphonium quaternaire Q⁺ est choisi dans le groupe formé par le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, le cation (hydroxy-2-éthyl)-1-méthyl-3-imidazolium, le cation (carboxy-2-éthyl)-1-méthyl-3-imidazolium, le diéthylpyrazolium, le N-butyl-N-méthylpyrrolidinium, le N-butyl-N-méthylmorpholinium, le triméthylphénylammonium, le tétrabutylphosphonium et le tributyl-tétradécylphosphonium

Les cations sulfonium quaternaires et guanidinium quaternaires répondent de préférence à l'une des formules générales :

SR¹R²R³⁺et C(NR¹R²)(NR³R⁴)(NR⁵R⁶)⁺

où R¹, R², R³, R⁴, R⁵ et R⁶, identiques ou différents, sont définis comme précédemment.

A titre d'exemples de sels utilisables selon l'invention, on peut citer le bis(trifluorométhylsulfonyl)amidure de butyl-3-méthyl-1-imidazolium, le bis(trifluorométhylsulfonyl)amidure de butyl-3-diméthyl-1,2-imidazolium, le bis(trifluorométhylsulfonyl)-amidure de N-butyl-N-méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, l'hexafluoroantimonate de butyl-3-méthyl-1-imidazolium, le trifluoroacétate de butyl-3-méthyl-1-imidazolium, le triflate d'éthyl-3-méthyl-1-imidazolium, le bis(trifluorométhylsulfonyl)amidure de (hydroxy-2-éthyl)-1-méthyl-3-imidazolium, le bis(trifluorométhylsulfonyl)amidure de (carboxy-2-éthyl)-1-méthyl-3-imidazolium et le bis(trifluorométhylsulfonyl)amidure de N-butyl-N-méthylmorpholinium. Ces sels peuvent être utilisés seuls ou en mélange.

### Les catalyseurs

Les catalyseurs utilisés dans le procédé de l'invention pour effectuer la réaction de métathèse des corps gras insaturés avec de l'éthylène en excès peuvent consister en tout catalyseur connu de métathèse et en particulier des catalyseurs comprenant au moins un composé du ruthénium.

Les catalyseurs au ruthénium sont choisis de préférence parmi les catalyseurs chargés ou non chargés de formule générale :

(X₁MX₂)_{b}Ru(carbène C)(L₁)_{c}(L₂)_{d}

dans laquelle :
- a, b, c, d sont des nombres entiers avec a et b égaux à 0, 1 ou 2 ; c et d égaux à 0, 1, 2, 3 ou 4;
- X₁ et X₂, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénates, les amidures, le tosylate, l'hexafluorophosphate, le tétrafluoroborate, le bis-triflylamidure, le tétraphénylborate et ses dérivés. X₁ ou X₂ peuvent être liés à L₁ ou L₂ ou au "carbène C" de façon à former un ligand bidenté (ou chélate) sur le ruthénium ; et
- L₁ et L₂, identiques ou différents, sont des ligands donneurs d'électrons tels que : phosphine, phosphite, phosphonite, phosphinite, arsine, stilbine, une oléfine ou un aromatique, un composé carbonylé, un ether, un alcool, une amine, une pyridine ou dérivé, une imine, un thioéthers, ou un carbène hétérocyclique qui répond par exemple à l'une des formules générales de la Figure 2, dans lesquelles R₁, R₂, R₃, R₄ et R₅, identiques ou différents, représentent chacun l'hydrogène, un groupe hydrocarboné, aliphatique, saturé ou insaturé, ou aromatique, comprenant de 1 à 12 atomes de carbone.

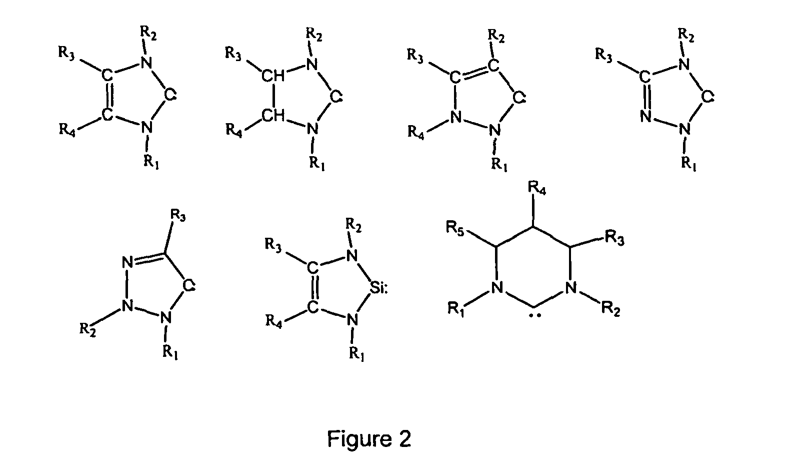

L₁ ou L₂ peuvent être liés au "carbène C " de façon à former un ligand bidenté ou chélaté, comme indiqué dans la formule (Figure 3) dans laquelle Z représente un bi-radical hydrocarboné, aliphatique, cyclique ou non, saturé ou insaturé, ou aromatique, comprenant de 1 à 12 atomes de carbone ; Y est un hétéroélément tel que l'oxygène, l'azote, le soufre ou le phosphore.

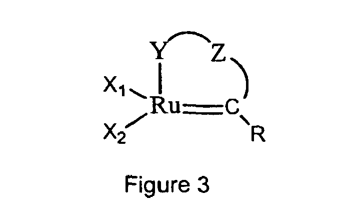

Le "carbène C" pourra être représenté par la formule générale : C(R₁)(R₂) pour laquelle R₁ et R₂ sont identiques ou différents tels que l'hydrogène ou tout groupe hydrocarbonyle, saturé ou insaturé, cyclique, branché ou linéaire, ou aromatique. A titre d'exemples, on pourra citer les complexes du ruthénium alkylidènes, ou cumulènes, tels que les vinylidènes Ru=C=CHR ou allénylidènes Ru=C=C=CR₁R₂ ou indénylidènes.

Un groupe fonctionnel permettant d'améliorer la rétention du complexe du ruthénium dans le liquide ionique peut être greffé sur au moins l'un des ligands X₁, X₂, L₁, L₂, ou sur le carbène C. Ce groupe fonctionnel peut être chargé ou non chargé, tel que de préférence un ester, un éther, un thiol, un acide, un alcool, une amine, un hétérocycle azoté, un sufonate, un carboxylate, un ammonium quaternaire, un guanidinium, un phosphonium quaternaire, un pyridinium, un imidazolium, un morpholinium ou un sulfonium.

Exemples de complexes fonctionnalisés : position possibles de la fonction

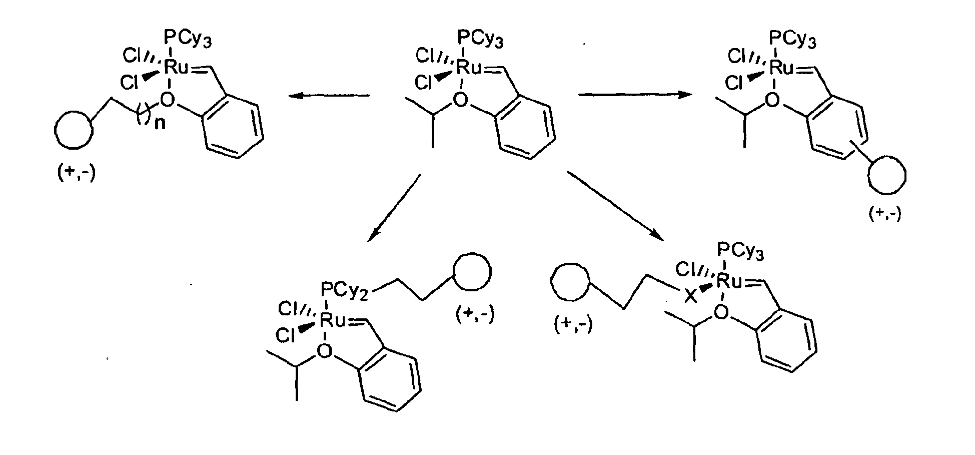

Parmi ces dérivés du ruthénium, on pourra citer les exemples suivants :

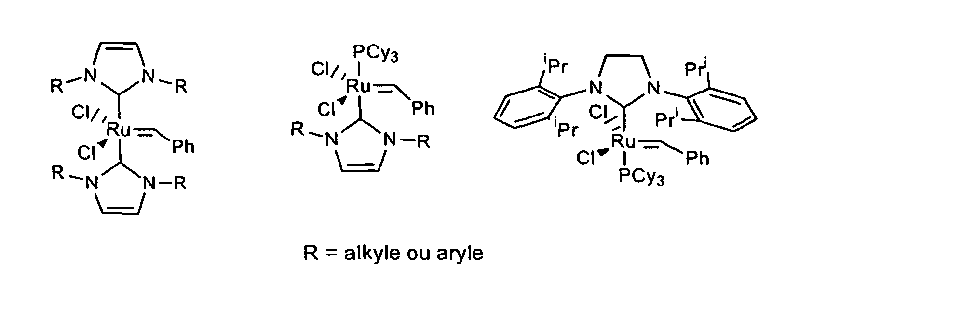

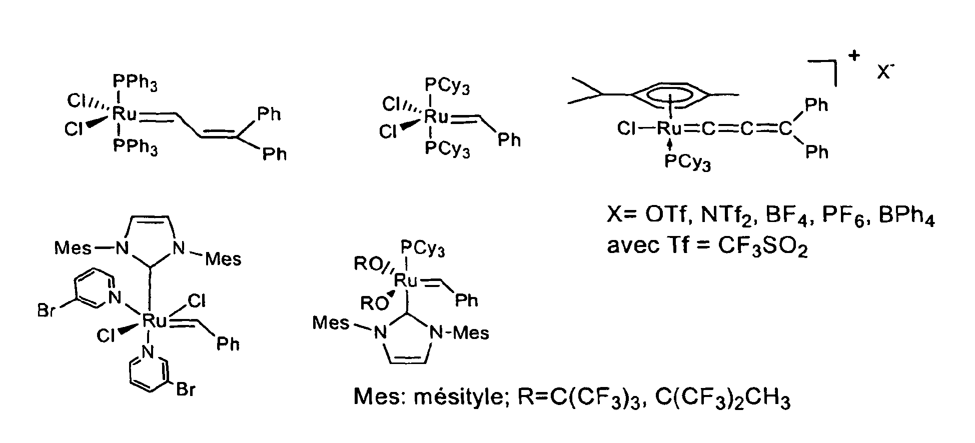

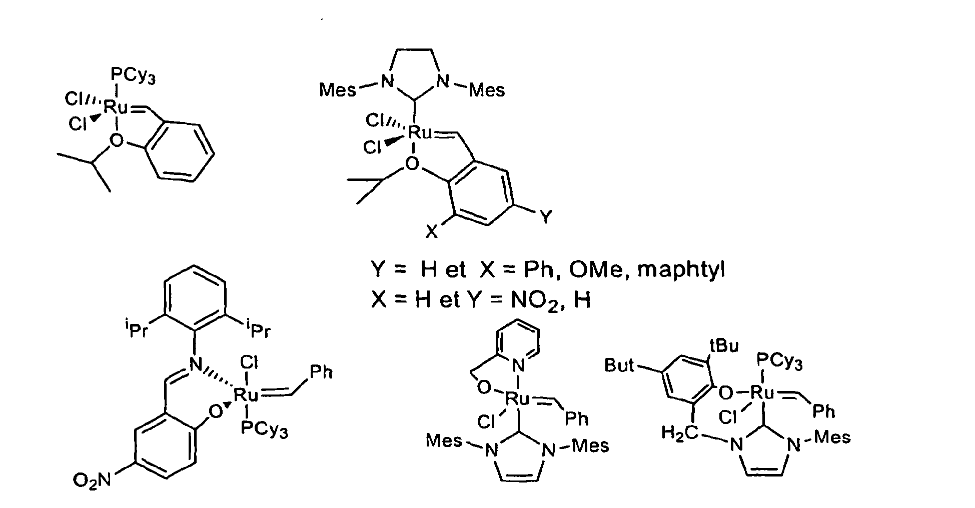

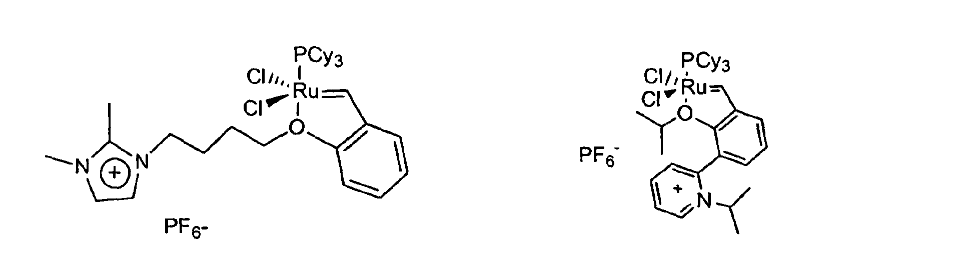

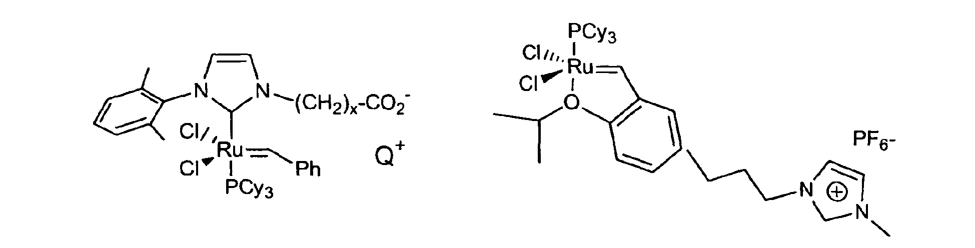

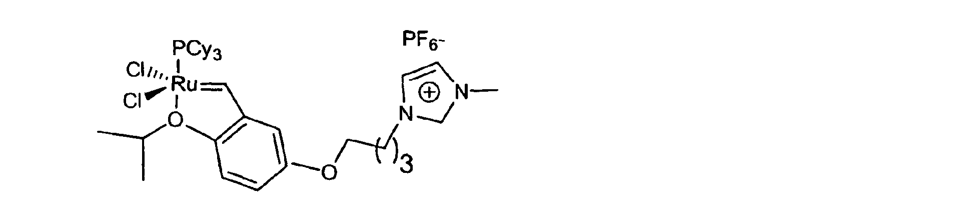

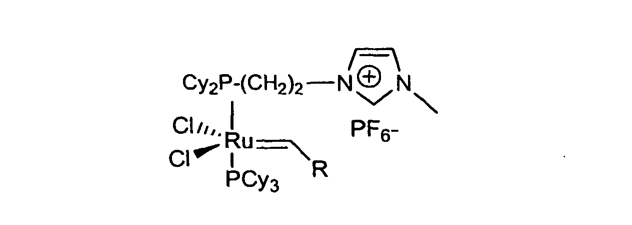

Dans ces formules Cy représente le radical cyclohexyle et iPr le radical isopropyle, Q⁺ représente un cation organique (par exemple ammonium, pyridinium, imidazolium ou phosphonium) ou inorganique (par exemple Na⁺, Li⁺ ou K⁺).

### La mise en oeuvre

Selon le procédé de l'invention, la réaction d'homométathèse du corps gras de départ (par exemple un ester de monoalcool de l'huile de tournesol oléique ou de l'huile de colza oléique) peut être conduite en l'absence ou en présence d'un co-solvant organique. Dans le cas où un solvant ou un mélange de solvants est utilisé, son rôle peut être d'améliorer la solubilisation des réactifs et du catalyseur dans le liquide ionique. II peut aussi servir à optimiser l'extraction des produits dans une deuxième phase.

Parmi les solvants envisageables pour l'invention, on peut citer par exemple les chloroalcanes, tels que le dichlorométhane, le chloroforme ou les dichloro- ou trichloroéthane, les solvants aromatiques tels que le toluène, les xylènes, ou le chlorobenzène, ou aliphatiques tels que l'heptane ou le cyclohexane.

Les réactions d'homométathèse selon le procédé de l'invention peuvent être conduites en système fermé (batch), en système semi-ouvert ou en continu avec un ou plusieurs étages de réaction. On peut également envisager de réaliser la réaction en utilisant une distillation réactive.

Une vigoureuse agitation doit assurer un bon contact entre les réactifs et le mélange catalytique. La température de réaction peut être de 0°C à +150°C, de préférence de 20°C à 120°C.

On peut opérer au-dessus ou en dessous de la température de fusion du milieu, l'état solide dispersé n'étant pas une limitation au bon déroulement de la réaction.

La pression peut aller par exemple de la pression atmosphérique à 50 MPa.

Les produits de la réaction peuvent être séparés par décantation.

Il est aussi possible de séparer les produits par distillation compte tenu de la non-volatilité du liquide ionique et de sa bonne stabilité thermique.

Les exemples suivants illustrent l'invention sans la limiter.

Exemple 1 : Homométathèse biphasique de l'oléate de méthyle dans un liquide ionique.

Dans un récipient en verre, on introduit 30 mg (0,036 mmol soit 0,004 éq.) d'un complexe au ruthénium benzylidène bis (tricyclohexylphosphine) dichlororuthénium, 3mL (8,84 mmol soit 1 éq.) d'oléate de méthyle, 1mL de liquide ionique bis(trifluorométhylsulfonyl)-amidure de 1-butyl-1-méthylpyrrolidinium de formule [BMPyrr][NTf₂] ainsi que 2 mL d'heptane et 0,1mL de dodécane (étalon interne pour la chromatographie). Le mélange est biphasique.

Le milieu est agité et chauffé à 55°C. Au bout d'un temps de réaction de 2 heures et décantation du mélange, un aliquote de la phase liquide supérieure est prélevé pour être analysé. Les analyses de chromatographie en phase gazeuse indiquent que la réaction de métathèse s'est déroulée de façon satisfaisante, les produits de la réaction étant le 9-octadécène et le diméthyloctadecène-1,18-dioate.

La conversion de l'oléate de méthyle est de 46% en poids.

### Exemple 2 : Tests de recyclage

Dans un récipient en verre, on introduit 50 mg (0,059 mmol, soit 0,01 éq.) d'un complexe au ruthénium 1,3-Bis-(2,4,6-trimethylphenyl)-2-imidazolidinylidene dichloro(benzylidene)(tricyclohexylphosphine) ruthenium, 1,5mL d'oléate de méthyle (4,42 mmol soit 1éq.), 1 mL de liquide ionique bis(trifluorométhylsulfonyl)-amidure de 1-butyl-1-méthylpyrrolidinium de formule [BMPyrr][NTf₂] ainsi que 2 mL d'heptane et 0,1mL de dodécane (étalon interne pour la chromatographie). Le mélange est biphasique.

Le milieu est agité à température ambiante. Au bout d'un temps de réaction de 2 heures, on laisse le mélange décanter. La phase supérieure est extraite et la solution de liquide ionique restante est lavée avec 2 mL d'heptane. Un aliquot du liquide organique est analysé par chromatographie en phase gazeuse (entrée 1)

A la phase liquide ionique, on ajoute 1,5 mL d'oléate de méthyle (4,42 mmol soit 1 éq.), 2mL d'heptane et 0,1 mL de docécane après chaque recyclage, ce qui permet à la réaction de redémarrer à température ambiante. Les analyses de chromatographie en phase gazeuse indiquent que la réaction de métathèse s'est déroulée de façon satisfaisante, les produits de la réaction étant principalement le 9-octadécène et le diméthyloctadecène-1,18-dioate.

Le recyclage de la phase ionique a été réalisé 3 fois successivement sans ajout intermédiaire de catalyseur au ruthénium, ni de liquide ionique.

La conversion de l'oléate de méthyle en 9-octadécène et en diméthyloctadecène-1,18-dioate est donnée dans le tableau 1.

**Table 1 : Conversion de l'oléate de méthyle en 9-octadécène et en diméthyloctadecène-1,18-dioate**

| **Entrée** | **Recyclage** | **Conversion (% pds)** |
|---|---|---|
| 1 | | 55 |
| 2 | 1^{er} | 54 |
| 3* | 2^{ème} | 50 |
| 4* | 3^{ème} | 50 |

| | | |
|---|---|---|
| *durée de réaction de15 heures. | | |

## Revendications

1. Procédé pour produire à la fois une fraction oléfinique et une composition de diesters ou de diacides **caractérisé en ce que** l'on soumet à une réaction d'homométathèse, au moins un corps gras comprenant au moins un monoacide carboxylique possédant de 12 à 22 atomes de carbone et comportant au moins une insaturation éthylénique ou un monoester d'un tel monoacide, en présence d'un catalyseur et en présence d'au moins un liquide ionique non-aqueux.

2. Procédé selon la revendication 1 **caractérisé en ce que** le liquide ionique non-aqueux est choisi dans le groupe formé par les sels liquides de formule générale Q⁺ A⁻ dans laquelle Q⁺ représente un phosphonium quaternaire, un ammonium quaternaire, un guanidinium quaternaire ou un sulfonium quaternaire et A⁻ représente tout anion susceptible de former un sel liquide en dessous de 90°C.

3. Procédé selon la revendication 2 **caractérisé en ce que** les cations Q⁺ ammonium ou phosphonium quaternaires, répondent à l'une des formules générales :
NR¹R²R³R⁴⁺ et PR¹R²R³R⁴⁺,
ou à l'une des formules générales
R¹R²N=CR³R⁴⁺ et R¹R²P=CR³R⁴⁺ dans lesquelles R¹, R², R³ et R⁴, identiques ou différents, représentent l'hydrogène (à l'exception du cation NH₄⁺ pour NR¹R²R³R⁴⁺), des radicaux hydrocarbyles ayant de 1 à 30 atomes de carbone ou des radicaux hydrocarbyles portant une ou plusieurs fonctions choisies parmi les fonctions -CO₂R, -C(O)R, -OR, -C(O)NRR',
-C(O)N(R)NR'R", -NRR', -SR, -S(O)R, -S(O)₂R, -SO₃R, -CN, -N(R)P(O)R'R', -PRR', -P(O)RR', -P(OR)(OR'), -P(O)(OR)(OR') dans lesquelles R, R' et R", identiques ou différents, représentent chacun l'hydrogène ou des radicaux hydrocarbyles ayant de 1 à 30 atomes de carbone.

4. Procédé selon la revendication 2 **caractérisé en ce que** les cations ammonium et/ou phosphonium quaternaires sont dérivés d'hétérocycles azotés et/ou phosphorés comportant 1, 2 ou 3 atomes d'azote et/ou de phosphore, de formules générales : 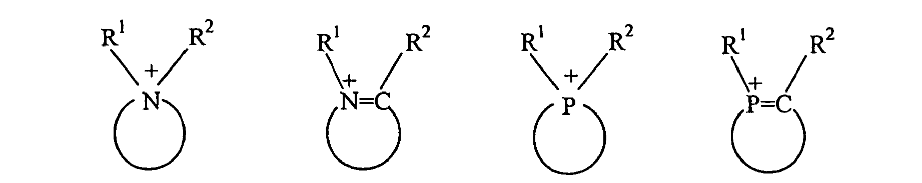 dans lesquelles les cycles sont constitués de 4 à 10 atomes, et R¹ et R², identiques ou différents, sont définis comme précédemment.

5. Procédé selon la revendication 2 **caractérisé en ce que** le cation ammonium ou phosphonium quaternaire répond à l'une des formules générales :
R¹R²⁺N=CR³-R⁷-R³C=N⁺R¹R² et R¹R²⁺P=CR³-R⁷-R³C=P⁺R¹R²
dans lesquelles R¹, R² et R³, identiques ou différents, sont définis comme précédemment, et R⁷ représente un radical alkylène ou phenylène.

6. Procédé selon l'une des revendications 2 à 5 **caractérisé en ce que** le cation ammonium et/ou phosphonium quaternaire Q⁺ est choisi dans le groupe formé par le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, le cation (hydroxy-2-éthyl)-1-méthyl-3-imidazolium, le cation (carboxy-2-éthyl)-1-méthyl-3-imidazolium, le diéthylpyrazolium, le N-butyl-N-méthylpyrrolidinium, le N-butyl-N-méthylmorpholinium le triméthylphénylammonium, le tétrabutylphosphonium et le tributyl-tétradécyl-phosphonium.

7. Procédé selon la revendication 2 **caractérisé en ce que** les cations sulfonium quaternaires et guanidinium quaternaires répondent à l'une des formules générales :
SR¹R²R³⁺ ou C(NR¹R²)(NR³R⁴)(NR⁵R⁶)⁺
où R1, R2, R3, R4, R5 et R6, identiques ou différents, sont définis comme R1, R2, R3 et R4 précédemment.

8. Procédé selon l'une des revendications 2 à 7 **caractérisé en ce que** les anions A⁻ sont choisis parmi les anions halogénures, nitrate, sulfate, alkylsulfates, phosphate, alkylphosphates, acétate, halogénoacétates, tétrafluoroborate, tétrachloroborate, hexafluorophosphate, trifluoro-tris-(pentafluoroéthyl) phosphate, hexafluoroantimonate, fluorosulfonate, alkylsulfonates, perfluoroalkylsulfonates, bis(perfluoroalkylsulfonyl)amidures, le méthylure de tris-trifluorométhylsulfonyle de formule C(CF3SO2)3-, le méthylure de bis-trifluorométhylsulfonyle de formule HC(CF3SO2)3⁻, arènesulfonates, arènesulfonates substitués par des groupements halogènes ou halogénoalkyles, l'anion tétraphenylborate et les anions tétraphenylborates dont les noyaux aromatiques sont substitués, tétra-(trifluoroacétoxy)-borate, bis-(oxalato)-borate, dicyanamide, tricyanométhylure, ainsi que l'anion tétrachloroaluminate, ou les anions chlorozincates.

9. Procédé selon l'une des revendications 2 à 7 **caractérisé en ce que** le liquide ionique est choisi parmi le bis(trifluorométhylsulfonyl)amidure de butyl-3-méthyl-1-imidazolium, le bis(trifluorométhylsulfonyl)amidure de butyl-3-diméthyl-1,2-imidazolium, le bis(trifluorométhylsulfonyl)amidure de N-butyl-N-méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, l'hexafluoroantimonate de butyl-3-méthyl-1-imidazolium, le trifluoroacétate de butyl-3-méthyl-1-imidazolium, le triflate de éthyl-3-méthyl-1-imidazolium, le bis(trifluorométhylsulfonyl)amidure de (hydroxy-2-éthyl)-1-méthyl-3-imidazolium, le bis(trifluorométhylsulfonyl)amidure de (carboxy-2-éthyl)-1-méthyl-3-imidazolium et le bis(trifluorométhylsulfonyl)amidure de N-butyl-N-méthylmorpholinium.

10. Procédé selon l'une des revendications 1 à 9 **caractérisé en ce que** ledit corps gras insaturé soumis à la réaction d'homométathèse comprend au moins un ester formé entre un acide monocarboxylique possédant de 12 à 22 atomes de carbone et comportant au moins une insaturation éthylénique et au moins un monoalcool aliphatique comportant de 1 à 8 atomes de carbone.

11. Procédé selon l'une des revendications 1 à 10 **caractérisé en ce que** ledit monoacide carboxylique est choisi parmi l'acide lauroléique, l'acide myristoléique, l'acide palmitoléique, l'acide oléique, l'acide gadoléique, l'acide cétoléique, l'acide cis-vaccénique, l'acide pétrosélinique, l'acide hypogéïque, l'acide gondoïque, l'acide érucique, l'acide nervonique, l'acide vaccénique, l'acide élaïdique, l'acide brassidique, l'acide undécylènique et l'acide ricinoléique.

12. Procédé selon l'une des revendications 10 à 11 **caractérisé en ce que** l'on soumet à la réaction de métathèse un corps gras insaturé choisi parmi les mélanges d'esters de monoalcool des huiles de tournesol oléiques et des huiles de colza oléiques, pour produire à la fois une fraction oléfinique et une composition de diesters de monoalcool dont au moins une partie des chaînes est constituée de chaînes insaturées en C18.

13. Procédé selon la revendication 12 **caractérisé en ce que** le corps gras insaturé est enrichi en acides gras monoinsaturés de type oléique par hydrogénation sélective d'acides gras contenant des acides polyinsaturés en C18.

14. Procédé selon la revendication 12 ou 13 **caractérisé en ce que** la composition en acides gras de l'huile de tournesol oléique a la composition suivante :
· oléate de méthyle (C18:1): environ 83 % en masse
· linoléate de méthyle (C18:2) : environ 10 % en masse
· palmitate de méthyle (C16:0) environ 3 % en masse et
· stéarate de méthyle (C18:0) : environ 4 % en masse.

15. Procédé selon l'une des revendications 1 à 14 **caractérisé en ce que** l'on utilise comme catalyseur au moins un composé du ruthénium.

16. Procédé selon la revendication 15 **caractérisé en ce que** le catalyseur est choisi parmi les catalyseurs chargés ou non chargés de formule générale :
(X₁)ₐ(X₂)_{b}Ru(carbène C)(L₁)_{c}(L₂)_{d}
dans laquelle :
• a, b, c, d sont des nombres entiers avec a et b égaux à 0, 1 ou 2 ; c et d égaux à 0, 1, 2, 3 ou 4;
• X₁ et X₂, identiques ou différents, représentent chacun un ligand mono- ou multi-chélatant, chargé ou non ; X₁ ou X₂ pouvant être liés à L₁ ou L₂ ou au carbène C de façon à former un ligand bidenté sur le ruthénium ; et
• L1 et L2 identiques ou différents sont des ligands donneurs d'électrons.

17. Procédé selon l'une des revendications 1 à 16 **caractérisé en ce qu'**il comprend en outre une étape dans laquelle on sépare :
· une fraction oléfinique ;
· et une composition de diesters de monoalcool ou de diacides.

18. Procédé selon l'une quelconque des revendications 1 à 17 **caractérisé en ce que** dans la fraction oléfinique obtenue par séparation plus de la moitié des chaînes est constituée de chaînes insaturées en C18.

19. Procédé selon la revendication 17 ou 18 **caractérisé en ce que** ladite fraction oléfinique comprend au moins 80 % d'octadéc-9-ène.

20. Procédé selon la revendication 19 **caractérisé en ce que** ladite fraction oléfinique comprend en outre du dodéc-6-ène, du pentadéc-6,9-diène, de l'octadéc-6,9-diène et de l'octadéc-6,9,-12-triène.

21. Procédé selon la revendication 17 **caractérisé en ce qu'**il comprend en outre une étape dans laquelle les monooléfines et les polyoléfines sont séparées de ladite fraction oléfinique par distillation.

22. Procédé selon l'une quelconque des revendications 1 à 17 **caractérisé en ce que** dans la composition de diesters de monoalcool ou de diacides obtenue par séparation plus de la moitié des chaînes est constituée de chaînes insaturées en C18.

23. Procédé selon la revendication 22 **caractérisé en ce que** dans ladite composition ledit monoalcool comporte de 1 à 8 atomes de carbone.

## Claims

1. A process for producing both an olefinic fraction and a composition of diesters or diacids, **characterized in that** it comprises carrying out a homometathesis reaction on at least one fat comprising at one monobasic carboxylic acid containing 12 to 22 carbon atoms and comprising at least one ethylenically unsaturated bond or a monoester of said monoacid in the presence of a catalyst and in the presence of at least one non-aqueous ionic liquid.

2. A process according to claim 1, **characterized in that** the non-aqueous ionic liquid is selected from the group formed by liquid salts with general formula Q⁺A⁻ in which Q⁺ represents a quaternary phosphonium, a quaternary ammonium, a quaternary guanidinium or a quaternary sulphonium and A⁻ represents any anion which is capable of forming a liquid salt below 90°C.

3. A process according to claim 2, **characterized in that** the cations Q⁺, quaternary ammonium or phosphonium, have one of the following formulae:
NR¹R²R³R⁴⁺ and PR¹R²R³R⁴⁺
or one of general formulae
R¹R²N=CR³R⁴⁺ and R¹R²P=CR³R⁴⁺
in which R¹, R², R³ and R⁴, which may be identical or different, represent hydrogen (with the exception of the cation NH₄⁺ for NR¹R²R³R⁴⁺), hydrocarbyl radicals containing 1 to 30 carbon atoms or hydrocarbyl radicals carrying one or more functions selected from -CO₂R, -C(O)R, -OR, -C(O)NRR', - C(O)N(R)NR'R", -NRR', -SR, -S(O)R, -S(O)₂R, -SO₃R, -CN, -N(R)P(O)R'R', - PRR', -P(O)RR', -P(OR)(OR'), -P(O)(OR)(OR') in which R, R' and R", which may be identical or different, each represent hydrogen or hydrocarbyl radicals containing 1 to 30 carbon atoms.

4. A process according to claim 2, **characterized in that** the quaternary ammonium and/or phosphonium cations may also be derived from nitrogen-containing and/or phosphorus-containing heterocycles comprising 1, 2 or 3 nitrogen and/or phosphorus atoms, with general formulae: 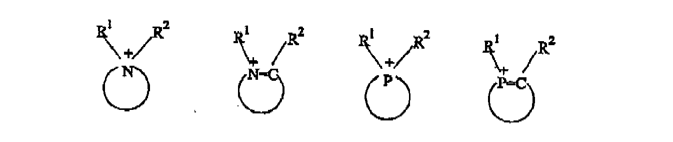 in which the cycles are constituted by 4 to 10 atoms and R¹ and R², which may be identical or different, are as defined above.

5. A process according to claim 2, **characterized in that** the quaternary ammonium or phosphonium cation has one of the following formulae:
R¹R²⁺N=CR³-R⁷-R³C=N⁺R¹R² and R¹R²⁺P=CR³-R⁷-R³C=P⁺R¹R²
in which R¹, R² and R³, which may be identical or different, are as defined above and R⁷ represents an alkylene or phenylene radical.

6. A process according to one of claims 2 to 5, **characterized in that** the quaternary ammonium and/or phosphonium cation Q⁺ is selected from the group formed by N-butylpyridinium, N-ethylpyridinium, pyridinium, 3-ethyl-1-methylimidazalium, 3-butyl-1-methylimidazolium, 3-hexyl-1-methylimidazolium, 3-hutyl-1,2-dimethylimidazolium, the 1-(2-hydroxyethyl)-3-methylimidazolium cation, the 1-(2-carboxyethyl)-3-methylimidazolium cation, diethylpyrazolium, N-butyl-N-methylpyrrolidinium, N-butyl-N-methylmorpholinium, trimethylphenylammonium, tetrabutylphosphonium and tributyltetradecylphosphonium.

7. A process according to claim 2, **characterized in that** the quaternary sulphonium and quaternary guanidinium cations have one of the following general formulae:
S R¹R²R³⁺ or C(NR¹R²)(NR³R⁴)(NR⁵R⁶)⁺
in which R¹, R², R³, R⁴, R⁵ and R⁶ , which may be identical or different, are defined as in R¹, R², R³ and R⁴ above.

8. A process according to one of claims 2 to 7, **characterized in that** the anions A⁻ are selected from the following anions: halides, nitrate, sulphate, alkylsulphates, phosphate, alkylphosphates, acetate, halogenoacetates, tetrafluoroborate, tetrachloroborate, hexafluorophosphate, trifluoro-tris-(pentafluoroethyl)phosphate, hexafluoroantimonate, fluorosulphonate, alkylsulphonates, perfluoroalkylsulphonates, bis(perfluoroalkylsulphonyl)amides, tristrifluoromethylsulphonyl methylide with formula C(CF₃SO₂)₃⁻, bistrifluoromethylsulphonyl mcthylide with formula HC(CF₃SO₂)₃⁻, arenesulphonates, arenesulphonates substituted with halogens or halogenalkyl groups, the tetraphenylborate anion and tetraphenylborate anions the aromatic rings of which are substituted, tetra-(trifluoroacetoxy)-borate, bis-(oxalato)-borate, dicyanamide, tricyanomethylide, as well as the tetrachloroaluminate anion, or chlorozincate anions.

9. A process according to one of claims 2 to 7, **characterized in that** the ionic liquid is selected from 3-butyl-1-methylimidazolium bis(trifluoromethylsulphonyl)amide, 3-butyl-1,2-dimethylimidazolium bis(trifluoromethylsulphonyl)amide, N-butyl-N-methylpyrrolidinium bis(trifluoromethylsulphonyl)amide, 3-butyl-1-methylimidazolium tetrafluoroborate, 3-butyl-1,2-dimethylimidazolium tetrafluoroborate, 3-ethyl-1-methylimidazolium tetrafluoroborate, 3-butyl-1-methylimidazolium hexafluoroantimonate, 3-butyl-1-methylimidazolium
trifluoroacetate, 3-ethyl-1-methylimidazolium triflate, 1-(2-hydroxyethyl)-3-methylimidazolium bis(trifluormethylsulphonyl)amide, 1-(2-carboxyethyl)-3-methylimidazolium bis(trifluoromethylsulphonyl)amide and N-butyl-N-methylmorpholinium bis(trifluoromethylsulphonyl)amide.

10. A process according to one of claims 1 to 9, **characterized in that** said unsaturated fat which undergoes the homometathesis reaction comprises at least one ester formed between a monocarboxylic acid containing 12 to 22 carbon atoms and comprising at least one ethylenically unsaturated bond and at least one aliphatic monoalcohol containing 1 to 8 carbon atoms.

11. A process according to one of claims 1 to 10, **characterized in that** said carboxylic monoacid is selected from lauroleic acid, myristoleic acid, palmitoleic acid, oleic acid, gadoleic acid, cetoleic acid, cis-vaccenic acid, petroselinic acid, hypogeic acid, gondoic acid, erucic acid, nervonic acid, vaccenic acid, elaidic acid, brassidic acid, undecylenic acid and ricinoleic acid.

12. A process according to claim 10 or claim 11, **characterized in that** an unsaturated fat selected from oleic mixtures of monoalcohol esters of oleic sunflower oil and oleic rapeseed oil undergoes the metathesis reaction to produce both an olefinic fraction and a monoalcohol diester composition at least a portion of the chains of which is constituted by unsaturated C₁₈ chains.

13. A process according to claim 12, **characterized in that** the unsaturated fat is enriched in oleic type monounsaturated fatty acids by selective hydrogenation of fatty acids containing C₁₈ polyunsaturated acids.

14. A process according to claim 12 or claim 13, **characterized in that** the composition of oleic sunflower oil fatty acids has the following composition:
• methyl oleate (C18:1): about 83% by weight;
• methyl linoleate (C18:2): about 10% by weight;
• methyl palmitate (C16:0): about 3% by weight;
• methyl stearate (C18:0): about 4% by weight.

15. A process according to one of claims 1 to 14, **characterized in that** at least one ruthenium compound is used as the catalyst.

16. A process according to claim 15, **characterized in that** the catalyst is selected from charged or uncharged catalysts with general formula:
(X₁)ₐ(X₂)_{b}Ru(carbene C)(L₁)_{c}(L₂)_{d} in which:
• a, b, c, d arc whole numbers in which a and b equal 0, 1 or 2; c and d equal 0, 1, 2, 3 or 4;
• X₁ and X₂, which may be identical or different, each represent a mono- or multi-chelating ligand, charged or uncharged; X₁ or X₂ may be bonded to L₁ or L₂ or to carbene C to form a bidentate ligand on the ruthenium; and
• L₁ and L₂, which may be identical or different, are donor electron ligands.

17. A process according to one of claims 1 to 16, **characterized in that** it further comprises a step in which the following arc separated:
• an olefinic fraction;
• and a composition of monoalcohol diesters or diacids.

18. A process according to any one of claims 1 to 17, **characterized in that** more than half of the chains in the olefinic fraction obtained by separation is constituted by unsaturated C₁₈ chains.

19. A process according to claim 17 or claim 18, **characterized in that** said olefinic fraction comprises at least 80% octadec-9-ene.

20. A process according to claim 19, **characterized in that** said olefinic fraction further comprises dodec-6-ene, pentadec-6,9-diene, octadec-6,9-diene and octadec-6,9,12-triene.

21. A process according to claim 17, **characterized in that** it further comprises a step in which the monoolefins and polyolefins are separated from said olefinic fraction by distillation.

22. A process according to any one of claims 1 to 17, **characterized in that** in the composition of monoalcohol diesters or diacids obtained by separation, more than half of the chains is constituted by unsaturated C₁₈ chains.

23. A process according to claim 22, **characterized in that** in said composition, said monoalcohol contains 1 to 8 carbon atoms.

## Patentansprüche

1. Verfahren zum gleichzeitigen Herstellen einer olefinischen Fraktion und einer Diester- oder Disäurezusammensetzung, **dadurch gekennzeichnet, dass** mindestens ein Fett, das mindestens eine Monocarbonsäure umfasst, die 12 bis 22 Kohlenstoffatome aufweist und mindestens eine ethylenische Ungesättigtheit oder einen Monoester einer solchen Monosäure umfasst, in Gegenwert eines Katalysators und in Gegenwart mindestens einer nichtwässrigen ionischen Flüssigkeit einer Homometathesereaktion unterzogen wird.

2. Verfahren nach Anspruch 1, **dadurch gekennzeichnet, dass** die nichtwässrige ionische Flüssigkeit ausgewählt ist aus der Gruppe gebildet aus den flüssigen Salzen mit der allgemeinen Formel Q⁺A⁻, worin Q⁺ für ein quartäres Phosphonium, ein quartäres Ammonium, ein quartäres Guanidinium oder ein quartäres Sulfonium steht und A⁻ für jedes Anion steht, das in der Lage ist, unterhalb von 90° C ein flüssiges Salz zu bilden.

3. Verfahren nach Anspruch 2, **dadurch gekennzeichnet, dass** die quartären Ammonium- oder Phosphoniumkationen Q⁺ einer der allgemeinen Formeln:
NR¹ R² R³R⁴⁺ und PR¹R²R³R⁴⁺,
oder einer der allgemeinen Formeln
R¹R²N=CR³R⁴⁺ und R¹R²P=CR³R⁴⁺
entsprechen, worin R¹ R², R³ und R⁴, gleich oder verschieden, für Wasserstoff (ausgenommen das Kation NH₄⁺ für NR¹R²R³R⁴⁺), Kohlenwasserstoffreste mit 1 bis 30 Kohlenstaffatomen oder Kohlenwasserstoffreste stehen, die eine oder mehrere Funktionen tragen, augewählt aus den Funktionen -CO₂R, -C(O)R, -OR, -C(O)NRR', -C(O)N(R)NR'R", - NRR', -SR, -S(O)R, -S(O)₂R, -SO₃R, -CN, -N(R)P(O)R'R', -PRR', - P(O)RR', -P(OR)(OR'), -P(O)(OR)(OR'), worin R, R' und R", gleich oder verschieden, jeweils für Wasserstoff oder Kohlenwasserstoffreste mit 1 bis 30 Kohlenstoffatomen stehen.

4. Verfahren nach Anspruch 2, **dadurch gekennzeichnet, dass** die quartären Ammonium- und/oder Phosphoniumkationen von stickstoffhaltigen und/oder phosphorhaltigen Heterocyclen abgeleitet sind, die 1, 2 oder 3 Stickstoff- und/oder Phosphoratome umfassen, mit den allgemeinen Formeln: 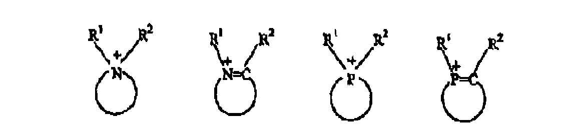 worin die Cyclen aus 4 bis 10 Atomen bestehen und R¹ und R², gleich oder verschieden, sind, wie oben definiert.

5. Verfahren nach Anspruch 2, **dadurch gekennzeichnet, dass** das quartäre Ammonium- oder Phosphoniumkation einer der folgenden allgemeinen Formeln entspricht:
R¹R²⁺N=CR³-R⁷-R³C=N⁺R¹R² und R¹R²⁺P=CR³-R⁷-R³C=P⁺R¹R²
worin R¹, R² und R³, gleich oder verschieden, sind, wie oben definiert, und R⁷ für einen Alkylen- oder Phenylenrest steht.

6. Verfahren nach einem der Ansprüche 2 bis 5, **dadurch gekennzeichnet, dass** das quartäre Ammonium- und/oder Phosphoniumkation Q⁺ ausgewählt ist aus der Gruppe gebildet aus N-Butylpyridinium, N-Ethylpyridinium, Pyridinium, Ethyl-3-methyl-1-imidazolium, Butyl-3-methyl-1-imidazolium, Hexyl-3-methyl-1-imidazolium, Butyl-9-dimethyl-1,2-imidazolium, (Hydroxy-2-ethyl)-1-methyl-3-imidazoliumkation, (Carboxy-2-ethyl)-1-methyl-3-imidazoliumkation, Diethylpyrazolium, N-Butyl-N-methylpyrrolidinium, N-Butyl-N-methylmorpholinium, Trimethylphenylammonium, Tetrabutylphosphonium und Tributyltetradecylphosphonium.

7. Verfahren nach Anspruch 2, **dadurch gekennzeichnet, dass** die quartären Sulfoniumkationen und die quartären Guanidiniumkationen einer der folgenden Formeln entsprechen:
SR¹R²R³⁺ oder C(NR¹R²)(NR³R⁴)(NR⁵R⁶)⁺
worin R¹, R², R³, R⁴, R⁵ und R⁶, gleich oder verschieden, sind, wie oben R¹, R², R³ und R⁴ definiert.

8. Verfahren nach einem der Ansprüche 2 bis 7, **dadurch gekennzeichnet, dass** die Anionen A⁻ ausgewählt sind aus den folgenden Anionen: Halogenid, Nitrat, Sulfat, Alkylsulfaten, Phosphat, Alkylphosphaten, Acetat, Halogenoacetaten, Tetrafluorborat, Tetrachlorborat, Hexafluorphosphat, Trifluor-tris(pentafluorethyl)phosphat, Hexafluorantimonat, Fluorsulfonat, Alkylsulfonaten, Perfluoralkylsulfonaten, Bis(perfluoralkylsulfonyl)amiden, Tristrifluormethylsulfonylmethylid mit der Formel C(CF₃SO₂)3⁻. Bistrifluormethylsulfonylmethylid mit der Formel HC(CF₃SO₂)3⁻, Arensulfonaten, Arensulfonaten, die mit Halogenen oder Hologenalkylgruppen substituiert sind, dem Tetraphenylboratanion und den Tetraphenylboratanlonen, deren aromatischen Kerne substituiert, sind. Tetra(trifluoracetoxy)borat, Bis-(oxalato)borat, Dicyanamid, Tricyanomethylid sowie dem Tetrschloraluminatanion oder den Chlorozinkatanionen.

9. Verfahren nach einem der Ansprüche 2 bis 7, **dadurch gekennzeichnet, dass** die ionische Flüssigkeit ausgewählt ist aus Butyl-3-methyl-1-imidazolium-bis(trifluormethylsulfonyl)amid, Butyl-3-dimethyl-1,2-imidazolium-bis(trifluormethylsulfonyl)amid, N-Butyl-N-methylpyrrolidinium-bis(trifluoromethylsulfonyl)amid, Butyl-3-methyl-1-imidazolium-tetrafluorborat, Butyl-3-dimethyl-1,2-imidazoliumtetrafluorborat, Ethyl-3-methyl-1-imidazolium-tetrafluoroborat, Butyl-3-methyl-1-Imidazolium-hexafluorantimonat, Butyl-3-methyl-1-imidazoliumtrifluoracetat, Ethyl-3-methyl-1-imidazolium-triflat, (Hydroxy-2-ethyl)-1-methyl-3-imidazolium-bis(trifluormethylsulfonyl)amid, (Garboxy-2-ethyl)-1-methyl-3-imidazolium-bis(trifluormethylsulfonyl)amid und N-Butyl-N-methylmorpholinium-bis(trifluormethylsulfonyl)amid.

10. Verfahren nach einem der Ansprüche 1 bis 8, **dadurch gekennzeichnet, dass** das ungesättigte Fett, das der Homometathesereaktion unterzogen wird, mindestens einen Ester umfasst, der zwischen einer Monocarbonsäure, die 12 bis 22 Kohlenstoffatome aufweist und mindestens eine ethylenische Ungesättigtheit umfasst, und mindestens einem aliphatischen Monoalkohol, der 1 bis 8 Kohlenstoffatome umfasst, gebildet wird.

11. Verfahren nach einem der Ansprüche 1 bis 10, **dadurch gekennzeichnet, dass** die Monocarbonsäure ausgewählt ist aus Lauroleinsäure, Myristoleinsäure, Palmitoleinsäure, Oleinsäure, Gadoleinsäure, Cetoleinsäure, cis-Vaccerisaure, Petroselinsäure, Hypogeinsäure, Gondosäure, Erucasäure, Nervonsäure, Vaccensäure, Elaidinsäure, Brassidinsäure, Undecylensäure und Ricinolsäure.

12. Verfahren nach einem der Ansprüche 10 bis 11, **dadurch gekennzeichnet, dass** ein ungesättigtes Fett, ausgewählt aus den Gemischen aus Monoalkoholestern der oleinischen Sonnenblumenöle und der oleinischen Rapsöle, der Metathesereaktion unterzogen wird, um gleichzeitig eine olefinische Fraktion und eine Monoalkoholdiesterzusammensetzung zu produzieren, bei der mindestens ein Teil der Ketten aus ungesättigten C18-Ketten besteht.

13. Verfahren nach Anspruch 12, **dadurch gekennzeichnet, dass** das ungesättigte Fett mit einfach ungesättigten Fettsäuren vom Oleinsäuretyp durch selektive Hydrierung der Fettsäuren, die mehrfach ungesättigte C18-Fettsäuren enthalten, angereichert wird.

14. Verfahren nach Anspruch 12 oder 13, **dadurch gekennzeichnet, dass** die Fettsaurezusammensetzung des oleinischen Sonnenblumenöls die folgende Zusammensetzung hat;
- Methyloleat (C18:1): etwa 83 Gew.-%
- Methylinoleat (C18:2): etwa 10 Gew.-%
- Methylpalmitat (C16:0): etwa 3 Gew.-% und
- Methylstearat (C18:0): etwa 4 Gew.-%.

15. Verfahren nach einem der Ansprüche 1 bis 14, **dadurch gekennzeichnet, dass** als Katalysator mindestens eine Rutheniumverbindung verwendet wird.

16. Verfahren nach Anspruch 15, **dadurch gekennzeichnet, dass** der Katalysator ausgewählt ist aus den beladenen oder nicht beladenen Katalysatoren, mit der allgemeinen Formel:
(X₁)ₐ(X₂)_{b}Ru(Carben C)(L₁)_{c}(L₂)_{d} worin:
- a, b, c, d ganze Zahlen sind, wobei a und b gleich 0, 1 oder 2; c und d gleich 0, 1, 2, 3 oder 4 sind;
- X₁ und X₂, gleich oder verschieden, jeweils für einen einfach oder mehrfach chelatbindenden Liganden, beladen oder nicht, stehen; X₁ oder X₂ an L₁ oder L₂ oder an das Carben C gebunden sein können, um einen zweizähligen Liganden auf dem Ruthenium bilden zu können; und
- L₁ und L₂, gleich oder verschieden, elekronenspendende Liganden sind.

17. Verfahren nach einem der Ansprüche 1 bis 16, **dadurch gekennzeichnet, dass** es ferner einen Schritt umfasst, indem:
- eine olefinische Fraktion;
- und eine Monoalkoholdiester- oder Disäurezusammensetzung getrennt werden.

18. Verfahren nach irgendeinem der Ansprüche 1 bis 17, **dadurch gekennzeichnet, dass** in der durch Trennung erhaltenen olefinischen Fraktion mehr als die Hälfte der Ketten aus ungesättigten C18-Ketten besteht.

19. Verfahren nach Anspruch 17 oder 18, **dadurch gekennzeichnet dass** die olefinische Fraktion mindestens 80 % Octadec-9-en umfasst.

20. Verfahren nach Anspruch 19, **dadurch gekennzeichnet, dass** die olefinische Fraktion ferner Dodec-6-en, Pentadec-6,9-dien, Octadec-6,9-dien und Octadec-6,9,-12-trien umfasst.

21. Verfahren nach Anspruch 17, **dadurch gekennzeichnet, dass** es ferner einen Schritt umfasst, in dem die Monoolefine und die Polyolefine von der olefinischen Fraktion durch Destillation getrennt werden.

22. Verfahren nach irgendeinem der Ansprüche 1 bis 17, **dadurch gekennzeichnet, dass** in der durch Trennung erhaltenen Monoalkoholdlester- oder Disäurezusammensetzung mehr als die Hälfte der Ketten aus ungesättigten C18-Ketten besteht.

23. Verfahren nach Anspruch 22, **dadurch gekennzeichnet, dass** in der Zusammensetzung der Monoalkohol 1 bis 8 Kohlenstoffatome umfasst.
